# EUROPEAN PATENT SPECIFICATION

(11) **EP 2 457 584 B1**
(45) Date of publication and mention of the grant of the patent: **09.08.2017**
(21) Application number: 11184754.7
(22) Date of filing: 01.06.2007
(51) Int. Cl.: C12N 7/00, A61K 35/76, A61K 35/768

(54) **MYXOMA VIRUS MUTANTS FOR CANCER TREATMENT**
MYXOMAVIRUS-MUTANTEN ZUR KREBSBEHANDLUNG
MUTANTS DU VIRUS MYXOMA POUR LE TRAITEMENT DU CANCER

(30) Priority: 01.06.2006 US 803640 P
(43) Date of publication of application: 30.05.2012
(62) Divisional of application: 07798014.2
(73) Proprietor: Robarts Research Institute, London, ON N6A 5K8 (CA)
(72) Inventor: BARRETT, John W., London, Ontario N6G 1W9 (CA); MCFADDEN, Grant, Gainesville, FL 32605 (US)
(74) Representative: Hobson, David James

(56) References cited:
- WO-A1-2004/078206
- JOHN W BARRETT ET AL: "Immunomodulatory proteins of myxoma virus", SEMINARS IN IMMUNOLOGY, vol. 13, no. 1, 1 February 2001 (2001-02-01), pages 73-84, XP55022515, ISSN: 1044-5323, DOI: 10.1006/smim.2000.0298
- CAMERON C ET AL: "The Complete DNA Sequence of Myxoma Virus", VIROLOGY, ACADEMIC PRESS,ORLANDO, US, vol. 264, no. 2, 25 November 1999 (1999-11-25), pages 298-318, XP004439702, ISSN: 0042-6822, DOI: 10.1006/VIRO.1999.0001
- LUN X ET AL: "Myxoma Virus is a Novel Oncolytic Virus with Significant Antitumor Activity against Experimental Human Gliomas", CANCER RESEARCH, AMERICAN ASSOCIATION FOR CANCER REREARCH, US LNKD- DOI:10.1158/0008-5472.CAN-05-1201, vol. 65, no. 21, 1 November 2005 (2005-11-01), pages 9982-9990, XP008120791, ISSN: 0008-5472
- BARRETT ET AL: "Myxoma virus M063R is a host range gene essential for virus replication in rabbit cells", VIROLOGY, ACADEMIC PRESS,ORLANDO, US, vol. 361, no. 1, 11 April 2007 (2007-04-11), pages 123-132, XP022029413, ISSN: 0042-6822, DOI: 10.1016/J.VIROL.2006.11.015

## Description

### BACKGROUND OF THE INVENTION

The use of certain genetically modified Myxoma viruses for treating cancer is disclosed in WO 04/078206 (Robarts Research Institute).

### SUMMARY OF THE INVENTION

The invention is as defined in the claims.

This invention relates to Myxoma viruses (MV) that are deficient in the activity of a Myxoma virus protein M063. Such viruses are for use in inhibiting a cancer cell *in vitro.* A method comprising administering to the cell an effective amount of the Myxoma virus is described herein. They are also for use in treating cancer in a human subject, and used in the manufacture of a medicament for treating cancer in a human subject. This invention also provides a pharmaceutical composition comprising such Myxoma viruses and a pharmaceutically acceptable carrier, as well as a kit comprising such Myxoma viruses and instructions for treating a cancer patient wherein the gene for Myxoma virus protein M063 is knocked out.

### DESCRIPTION OF THE FIGURES

Figure 1. Endogenous activated Akt levels in human glioma cells
Figure 2. Viral replication efficiency of the various vMyx-hrKOs and controls in human glioma cell lines.
Figure 3. Secreted early and late viral gene expression indicates that some of the vMyx-hrKO are unable to transit from early to late gene expression.
Figure 4. Selected single step growth curves.
Figure 5. Cell-based cytotoxicity assay

### DETAILED DESCRIPTION OF THE INVENTION

WO 04/078206 (Robarts Research Institute) discloses the use of certain genetically modified myxoma viruses for treating cancer. This invention represents an advance by providing more specific modified myxoma viruses for such uses. The techniques disclosed therein are applicable generally to the myxoma viruses of this invention.

As used herein "deficient in the activity of" a given Myxoma virus protein means that the virus has less of the activity in question than wild-type Myxoma virus. "Substantially no activity" of a given viral protein means that the virus has no detectable level of such activity. Examples of Myxoma viruses having substantially no activity of a given viral protein include mutants in which the gene for such protein has been deleted or otherwise knocked-out.

In accordance with this disclosure, any kind of cancer or cancer cell can be inhibited or treated. A cancer cell which is a mammalian cancer cell is described herein. In a more specific embodiment, the cancer cell is a human cancer cell. Examples of such human cancer cells include gliomas.

It has been demonstrated that wild-type myxoma virus (vMyxgfp) can produce a productive, long-lived infection, and destroy and clear implanted tumor tissue when injected intratumorally into human gliomas implanted in murine brains (Lun et al, 2005 Cancer Research 65:9982-9990). As well, a screen of the NCI-60 reference collection indicated that MV productively infects the majority (15/21) of human tumor cells tested (Sypula et al. 2004 Gene Ther. Mol. Biol. 8: 103-114). To expand understanding of MV tropism in cancer cells, a series of human glioma cells (U87, U118, U251, U343, U73) that were previously tested for wild-type MV permissiveness were screened. These findings have been extended in the following Examples by testing the infection and replication of several MV viruses in which specific host range genes, identified as having a role in defining MV tropism in rabbit cells, have been deleted. These viruses are collectively referred to as host range knockouts (vMyx-hrKO). Variation was observed in the ability of various vMyx-hrKOs to replicate and spread in the human glioma cells. vMyxT2(U251), vMyxT4KO (U87, U118, U251 and U373) and vMyxT5KO (U251, U373) exhibited some restriction in specific human gliomas. In contrast vMyx63KO and vMyx135KO appeared to replicate and kill more effectively in several of the gliomas.

The invention will be better understood by reference to the following examples, which illustrate but do not limit the invention described herein.

### EXAMPLES

### EXAMPLE 1

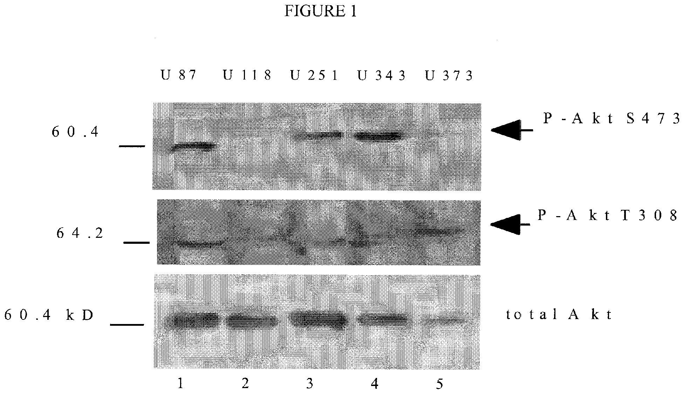

Fifty micrograms of whole cell lysates were probed with antibodies against phosphorylated Akt at positions threonine 308 (P-Akt T308) and serine 473 (P-Akt S473) or total Akt. Based on the levels of activated Akt U87 and U343 would be expected to be infectable and U118, U251 and U373 to be more resistant to MV infection. (Figure 1).

### EXAMPLE 2

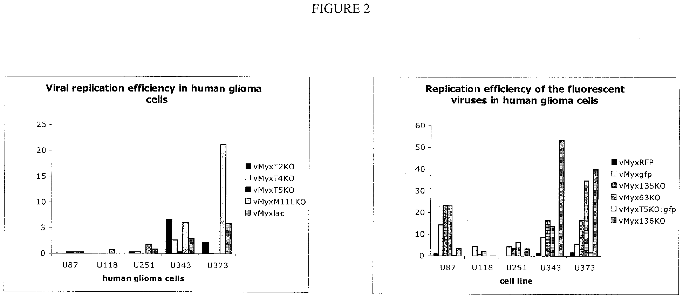

Virus stocks were titrated on the various glioma cells and control BGMK or RK13 cells. Virus titres, derived from the gliomas, were compared to the control levels and a value of viral replication efficiency was estimated. Based on these results none of the gliomas supported viral growth to the levels observed in the control lines. However some viruses (vMyx135KO, vMyx63KO and vMyx136KO) were more efficient than other knockouts. As well, some glioma lines supported more replication (U87, U343 and U373). (Figure 2)

### EXAMPLE 3

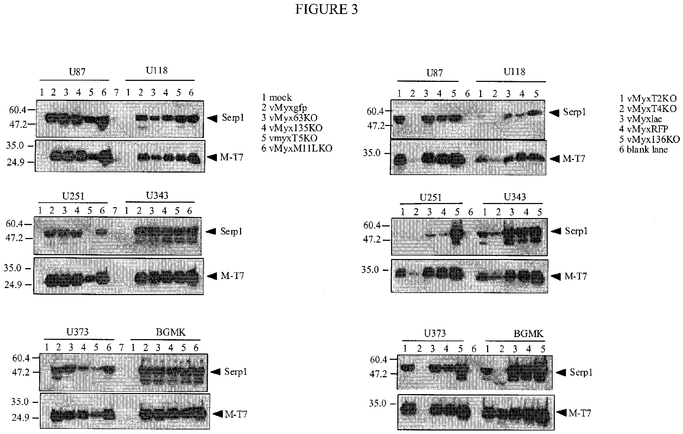

Various human gliomas were infected with a range of vMyx-hrKOs. Twenty hpi the infected supernatants were collected and concentrated 10x. Fifteen microlitres of concentrated sups were separated on a 12% SDS-PAGE gel and probed with anti-Serp1 (mAB; late MV gene product). The blots were stripped and probed for early gene expression with anti-M-T7 (pAB; early MV gene product). The results suggest that several vMyx-hrKOs are restricted in their transit from early to late gene expression including vMyxT2KO, vMyxT4KO and vMyxT5KO. And in two glioma lines (U87 and U37), vMyxT4KO does not even undergo early gene expression. (Figure 3).

### EXAMPLE 4

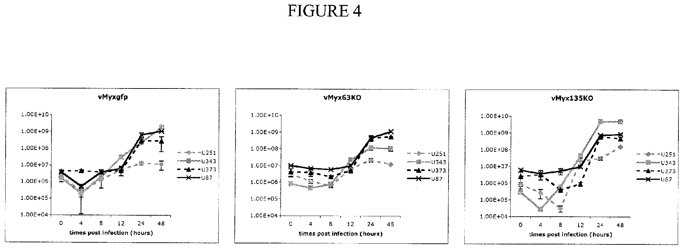

Cells were seeded in 48 well dishes and infected cells were collected at the times indicated. Virus was released from the collected cell pellets and titrated back onto BGMK cells. Although there was replication of the tested viruses, the best amplification appeared to occur in the U87 and U343 cells. (Figure 4).

### EXAMPLE 5

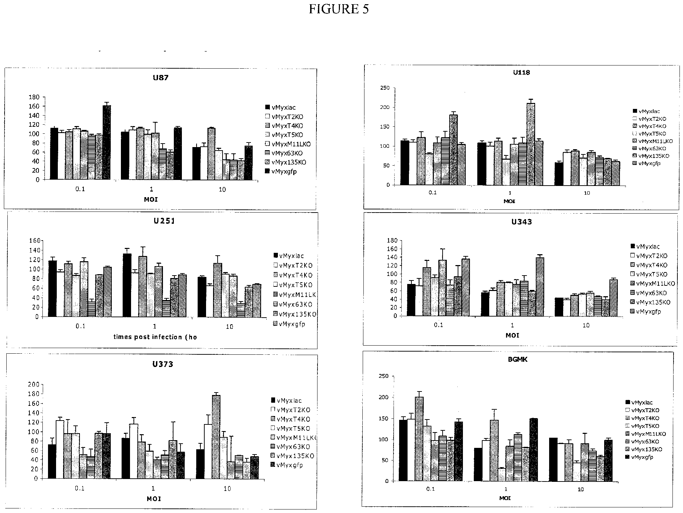

The ability of the various vMyx-hrKOs and control viruses to have a killing effect in the panel of human gliomas was tested by a cytotoxicity assay. The appropriate cells were seeded in 96 well dishes and 24 h later were infected with the viruses at various MOIs. Seventy-two hours post infection the infected cells were treated with the WTS reagent (Roche) to measure cell viability. Colour changes were measured at 450nm every 60 minutes for 4 hours. Uninfected control wells were used to determine normal proliferation and a blank well served as a background control. (Figure 5).

### CLAUSES

Clause 1. A method for inhibiting a cancer cell comprising administering to the cell an effective amount of a Myxoma virus that is deficient in the activity of a Myxoma virus protein selected from the group consisting of M11L, M063, M136, M-T4 and M-T7.
Clause 2. The method of clause 1, wherein the Myxoma virus has substantially no activity of the Myxoma virus protein selected from the group consisting of M11L, M063, M136, M-T4 and M-T7.
Clause 3. The method of clause 1, wherein the cell is a mammalian cancer cell.
Clause 4. The method of clause 3, wherein the cell is a human cancer cell.
Clause 5. The method of clause 4, wherein the cell is a glioma cell.
Clause 6. A method for treating a human subject having cancer, comprising administering to the patient an effective amount of a Myxoma virus that is deficient in the activity of a Myxoma virus protein selected from the group consisting of M11L, M063, M136, M-T4 and M-T7.
Clause 7. The method of clause 6, wherein the Myxoma virus has substantially no activity of the Myxoma virus protein selected from the group consisting of M11L, M063, M136, M-T4 and M-T7.
Clause 8. The method of clause 6, wherein the cancer is a glioma.
Clause 9. Use of an effective amount of a Myxoma virus for inhibiting a cancer cell, wherein the Myxoma virus is deficient in the activity of a Myxoma virus protein selected from the group consisting of M11L, M063, M136, M-T4 and M-T7.
Clause 10. Use of an effective amount of a Myxoma virus in the manufacture of a medicament for inhibiting a cancer cell, wherein the Myxoma virus is deficient in the activity of a Myxoma virus protein selected from the group consisting of M11L, M063, M136, M-T4 and M-T7.
Clause 11. The use of clause 9 or 10, wherein the Myxoma virus has substantially no activity of the Myxoma virus protein selected from the group consisting of M11L, M063, M136, M-T4 and M-T7.
Clause 12. The use of clause 9 or 10, wherein the cell is a mammalian cancer cell.
Clause 13. The use of clause 12, wherein the cell is a human cancer cell.
Clause 14. The use of clause 13, wherein the cell is a glioma cell.
Clause 15. Use of an effective amount of a Myxoma virus for treating a human subject having cancer, wherein the Myxoma virus is deficient in the activity of a Myxoma virus protein selected from the group consisting of M11L, M063, M136, M-T4 and M-T7.
Clause 16. Use of an effective amount of a Myxoma virus in the manufacture of a medicament for treating a human subject having cancer, wherein the Myxoma virus is deficient in the activity of a Myxoma virus protein selected from the group consisting of M11L, M063, M136, M-T4 and M-T7.
Clause 17. The use of clause 15 or 16, wherein the Myxoma virus has substantially no activity of the Myxoma virus protein selected from the group consisting of M11L, M063, M136, M-T4 and M-T7.
Clause 18. The use of clause 15 or 16, wherein the cancer is a glioma.
Clause 19. A pharmaceutical composition comprising a Myxoma virus and a pharmaceutically acceptable carrier for use in treating cancer, wherein the Myxoma virus is deficient in the activity of a Myxoma virus protein selected from the group consisting of M11L, M063, M136, M-T4 and M-T7.
Clause 20. The pharmaceutical composition of clause 19, wherein the Myxoma virus has substantially no activity of the Myxoma virus protein selected from the group consisting of M11L, M063, M136, M-T4 and M-T7.
Clause 21. The pharmaceutical composition of clause 19, wherein the cancer is a glioma.
Clause 22. A kit comprising a Myxoma virus and instructions for treating a patient having cancer, wherein the Myxoma virus is deficient in the activity of a Myxoma virus protein selected from the group consisting of M11L, M063, M136, M-T4 and M-T7.
Clause 23. The kit of clause 22, wherein the Myxoma virus has substantially no activity of the Myxoma virus protein selected from the group consisting of M11L, M063, M136, M-T4 and M-T7.

## Claims

1. A Myxoma virus in which the gene for Myxoma virus protein M063 is knocked out, for inhibiting a cancer cell *in vitro.*

2. Use of a Myxoma virus in which the gene for Myxoma virus protein M063 is knocked out, for inhibiting a cancer cell *in vitro.*

3. The Myxoma virus according to Claim 1 or use according to Claim 2, wherein the cell is a mammalian cancer cell.

4. The Myxoma virus or use according to Claim 3, wherein the cell is a human cancer cell.

5. The Myxoma virus or use according to Claim 4, wherein the cell is a glioma cell.

6. A Myxoma virus in which the gene for Myxoma virus protein M063 is knocked out, for use in treating cancer in a human subject.

7. Use of a Myxoma virus in which the gene for Myxoma virus protein M063 is knocked out, in the manufacture of a medicament for treating cancer in a human subject.

8. The Myxoma virus for use according to Claim 6 or use according to Claim 7, wherein the cancer is a glioma.

9. A pharmaceutical composition comprising a Myxoma virus and a pharmaceutically acceptable carrier, wherein the gene for Myxoma virus protein M063 is knocked out.

10. A pharmaceutical composition comprising a Myxoma virus and a pharmaceutically acceptable carrier for use in treating cancer, wherein the gene for Myxoma virus protein M063 is knocked out.

11. Use of a pharmaceutical composition comprising a Myxoma virus and a pharmaceutically acceptable carrier in the manufacture of a medicament for treating cancer, wherein the gene for Myxoma virus protein M063 is knocked out.

12. The pharmaceutical composition for use according to Claim 10 or use according to Claim 11, wherein the cancer is a glioma.

13. A kit comprising a Myxoma virus and instructions for treating a patient having cancer, wherein the gene for Myxoma virus protein M063 is knocked out.

## Patentansprüche

1. Myxomvirus, in dem das Gen für das Myxomvirus-Protein M063 abgeschaltet (knocked out) ist, zur Inhibition einer Krebszelle *in vitro.*

2. Verwendung eines Myxomvirus, in dem das Gen für das Myxomvirus-Protein M063 abgeschaltet (knocked out) ist, zur Inhibition einer Krebszelle *in vitro.*

3. Myxomvirus nach Anspruch 1 oder Verwendung nach Anspruch 2, wobei die Zelle eine Säugerkrebszelle ist.

4. Myxomvirus oder Verwendung nach Anspruch 3, wobei die Zelle eine humane Krebszelle ist.

5. Myxomvirus oder Verwendung nach Anspruch 4, wobei die Zelle eine Gliomzelle ist.

6. Myxomvirus, in dem das Gen für das Myxomvirus-Protein M063 abgeschaltet (knocked out) ist, zur Verwendung bei der Behandlung von Krebs bei einem humanen Patienten.

7. Verwendung eines Myxomvirus, in dem das Gen für das Myxomvirus-Protein M063 abgeschaltet (knocked out) ist, bei der Herstellung eines Arzneimittels zur Behandlung von Krebs bei einem humanen Patienten.

8. Myxomvirus zur Verwendung nach Anspruch 6 zur Verwendung nach Anspruch 7, wobei der Krebs ein Gliom ist.

9. Pharmazeutische Zusammensetzung, umfassend ein Myxomvirus und einen pharmazeutisch verträglichen Träger, wobei das Gen für das Myxomvirus-Protein M063 abgeschaltet (knocked out) ist.

10. Pharmazeutische Zusammensetzung, umfassend ein Myxomvirus und einen pharmazeutisch verträglichen Träger zur Verwendung bei der Behandlung von Krebs, wobei das Gen für das Myxomvirus-Protein M063 abgeschaltet (knocked out) ist.

11. Verwendung einer pharmazeutischen Zusammensetzung, umfassend ein Myxomvirus und einen pharmazeutisch verträglichen Träger bei der Herstellung eines Arzneimittels zur Behandlung von Krebs, wobei das Gen für das Myxomvirus-Protein M063 abgeschaltet (knocked out) ist.

12. Pharmazeutische Zusammensetzung zur Verwendung nach Anspruch 10 oder Verwendung nach Anspruch 11, wobei der Krebs ein Gliom ist.

13. Kit, umfassend ein Myxomvirus und Anleitungen zur Behandlung eines Patienten mit Krebs, wobei das Gen für das Myxomvirus-Protein M063 abgeschaltet (knocked out) ist.

## Revendications

1. Virus Myxoma, dans lequel le gène pour la protéine M063 du virus Myxoma est inactivé par knock-out, destiné à inhiber une cellule cancéreuse in vitro.

2. Utilisation d'un virus Myxoma dans lequel le gène pour la protéine M063 du virus Myxoma est inactivé par knock-out, pour inhiber une cellule cancéreuse in vitro.

3. Virus Myxoma selon la revendication 1 ou utilisation selon la revendication 2, dans lequel ou dans laquelle la cellule est une cellule cancéreuse mammifère.

4. Virus Myxoma ou utilisation selon la revendication 3, dans lequel ou dans laquelle la cellule est une cellule cancéreuse humaine.

5. Virus Myxoma ou utilisation selon la revendication 4, dans lequel ou dans laquelle la cellule est une cellule de gliome.

6. Virus Myxoma, dans lequel le gène pour la protéine M063 du virus Myxoma est inactivé par knock-out, destiné à une utilisation pour traiter un cancer chez un sujet humain.

7. Utilisation d'un virus Myxoma dans lequel le gène pour la protéine M063 du virus Myxoma est inactivé par knock-out, dans la fabrication d'un médicament destiné à traiter un cancer chez un sujet humain.

8. Virus Myxoma destiné à une utilisation selon la revendication 6 ou utilisation selon la revendication 7, dans lequel ou dans laquelle le cancer est un gliome.

9. Composition pharmaceutique comprenant un virus Myxoma et un support pharmaceutiquement acceptable, dans laquelle le gène pour la protéine M063 du virus Myxoma est inactivé par knock-out.

10. Composition pharmaceutique comprenant un virus Myxoma et un support pharmaceutiquement acceptable, destinée à une utilisation pour traiter un cancer, dans laquelle le gène pour la protéine M063 du virus Myxoma est inactivé par knock-out.

11. Utilisation d'une composition pharmaceutique comprenant un virus Myxoma et un support pharmaceutiquement acceptable dans la fabrication d'un médicament destiné à traiter un cancer, dans laquelle le gène pour la protéine M063 du virus Myxoma est inactivé par knock-out.

12. Composition pharmaceutique destinée à une utilisation selon la revendication 10 ou utilisation selon la revendication 11, dans laquelle le cancer est un gliome.

13. Trousse comprenant un virus Myxoma et des instructions pour traiter un patient atteint d'un cancer, dans laquelle le gène pour la protéine M063 du virus Myxoma est inactivé par knock-out.
